# EUROPEAN PATENT SPECIFICATION

(11) **EP 2 770 321 B1**
(45) Date of publication and mention of the grant of the patent: **11.04.2018**
(21) Application number: 12841113.9
(22) Date of filing: 16.05.2012
(51) Int. Cl.: G01N 21/76, G01N 33/15, G01N 33/52

(54) **METHOD FOR DETERMINING THE QUANTITY AND/OR ACTIVITY OF ANTIOXIDANTS**
VERFAHREN ZUR BESTIMMUNG DER MENGE UND/ODER AKTIVITÄT VON ANTIOXIDANTIEN
PROCÉDÉ POUR DÉTERMINER LA QUANTITÉ ET/OU L'ACTIVITÉ DES ANTIOXYDANTS

(30) Priority: 21.10.2011 EA 201101629
(43) Date of publication of application: 27.08.2014
(73) Proprietor: Disoft, Limited Liability Company, Moscow 119991 (RU)
(72) Inventor: IZMAYLOV, Dmitry Yurievich, Obninsk Kaluzhskaya obl. 249030 (RU); VLADIMIROV, Jury Andreevich, Moscow 117607 (RU)
(74) Representative: Lapienis, Juozas
(86) International application number: PCT/RU2012/000387
(87) International publication number: WO 2013/058676

(56) References cited:
- YU. A. VLADIMIROV ET AL: "Dihydroquercetin (taxifolin) and other flavonoids as inhibitors of free radical formation at key stages of apoptosis", BIOCHEMISTRY (MOSCOW), vol. 74, no. 3, 1 March 2009 (2009-03-01), pages 301-307, XP055184395, ISSN: 0006-2979, DOI: 10.1134/S0006297909030092
- YU. A. VLADIMIROV ET AL: "Free radicals and cell chemiluminescence", BIOCHEMISTRY (MOSCOW), vol. 74, no. 13, 1 December 2009 (2009-12-01), pages 1545-1566, XP055184393, ISSN: 0006-2979, DOI: 10.1134/S0006297909130082
- KLEBANOV G. . ET AL.: 'Antioksidantnye svoistva proizvodnykh 3-oksipiridina: meksidola, emoksipina i proksipina.' VOPROSY MEDITSINSKOI KHIMII 0042-8809 vol. T. 47, no. 3, 2001, pages 288 - 300, XP008173505
- VLADIMIROV JU.A. ET AL. SVOBODNYE RADIKALY I KLETOCHNAYA IJUMINESTSENTSIYA.USPEKHI BIOLOGICHESKOI KHIMII vol. 49, 2009, pages 341 - 388, XP008173506
- TRUBNIKOVA JU.N ET AL.: 'Issledovanie antioksidantnoi aktivnosti nekotorykh rastitelnykh balzamov. Zhurnal' MEDITSINA I OBRAZOVANIE V SIBIRI 2009, XP055154386 Retrieved from the Internet: <URL:http://www.ngmu.ru/cozo/mos/article/te xt-full.php?id=372>

## Description

### Field of Invention

The invention relates to analytical chemistry; particularly, it relates to means for determining amount and/or activity of antioxidants in test samples.

### Background of Invention

Methods used for assaying the antioxidant capacity (i.e. the total number of free radicals which can undergo a reaction with an antioxidant substance) are well known in the art and they may be conditionally categorized into a few groups including spectrophotometric methods, electrochemical methods, chemiluminescent methods and others methods.

Spectrophotometric methods of assaying the antioxidant capacity of samples are based on measuring the quantitative change in optical density of a reaction mixture in which oxidation-reduction reactions occur upon adding a test sample (see e.g. Chinese applications No 101413896, 101718703, 101241076 and Mexican application No 2008011324). The measurement results are expressed in relative and non-standardized units (which standardized units are: mole for amount of a substance and molarity for concentration of a substance), usually in trolox equivalents or other reference (i.e. commonly accepted) antioxidant equivalents.

Electrochemical methods used in assaying antioxidants are based on measuring quantitative change in voltage-current characteristics of oxidation-reduction reactions upon adding a test sample to the reaction mixture (see e.g. Russian patent No 2238555). A limitation of these methods consists in that the oxidation processes occur under the action of external electric force, i.e. in a forcible manner. As a result, the antioxidant properties of substances under the measurement conditions do not correspond to the antioxidant properties of the substances under natural conditions.

Chemiluminescent methods in biology and medicine are generally described in the article of Yu. A. Vladimirov ET AL "Free Radicals and Cell Chemiluminescence", Biochemistry (Moscow), vol. 74, no. 13, 1 December 2009 (2009-12-01), pages 1545-1566. Another article of Yu. A. Vladimirov ET AL "Dihydroquercetin (taxifolin) and other flavonoids as inhibitors of free radical formation at key stages of apoptosis", Biochemistry (Moscow), vol. 74, no. 3, 1 March 2009 (2009-03-01), pages 301-307, describes a more specific study of effect of antioxidants on the process of free radical formation. However, this document does not address a problem of determining any characteristics of antioxidants, and studies only effect thereof on chemiluminiscence intensity. Meanwhile, chemiluminescent methods of assaying the antioxidant capacity make it possible to directly detect free radicals, not oxidation products (see, for example, Russian patent No 2206891). These methods allow capturing kinetics of interaction between radicals and antioxidants i.e. registering natural progress with time of a process. A limitation of chemiluminescent methods consists in that reliable determination of amount and activity of an antioxidant in a test sample is not possible. This is due to the fact that all prior art chemiluminescent methods use indirect (amplitude, temporal or mixed) chemiluminescence kinetic parameters, based on which it is impossible to simulate adequately the processes that take place in the reaction mixture (see also patent publications GB 2245062 A and DE 10327971).

Thus, common limitations of the prior art methods reside in that the measurement results are relative in their nature and they are expressed via comparison of parameters of a test sample with parameters of a reference sample containing a predetermined amount of a reference substance. The antioxidant capacity values depend on the reference substance, which makes it impossible to compare the antioxidant capacity of substances whose properties have been determined in comparison with different reference substances. Moreover, the prior art methods do not allow determining amount of an antioxidant independently of its activity. Therefore, it can not be ascertained which factors (the amount of an antioxidant or the activity thereof) are responsible for the antioxidant capacity of a sample.

### Summary of Invention

The terms and expressions used herein have the following meaning, unless the context indicates otherwise.

An antioxidant compound (an antioxidant) is an individual chemical compound which is able to interact with free radicals, thereby inhibiting accumulation of oxidation products. Antioxidant capacity is the number of radicals which an antioxidant may come into interaction with, independently of the amount of radicals coming into separate interaction with each molecule of the antioxidant.

Antioxidant activity is ability of a substance to interact with free radicals, characterized by the value of the reaction rate constant of the corresponding reaction.

A strong antioxidant is a substance characterized by a comparatively high value of the reaction rate constant for interaction with free radicals.

A weak antioxidant is a substance characterized by a comparatively low value of the reaction rate constant for interaction with free radicals.

An activator (a chemiluminescence-enhancing reagent) is a substance emitting a quantum of electromagnetic radiation, mainly in the visible part of spectrum, upon interacting with a free radical, and having high quantum efficiency.

A source of free radicals is a substance which generates free radicals species upon decomposition of this substance, or upon reaction with other substances.

Radiation intensity is the number of photons passing through a unit of area per a unit of time. Other terms and expressions are used in their usual meaning intrinsic to the context and to the field of the invention.

The object of the present invention is to overcome the limitations of the prior art methods and to provide a new purpose-specific means (i.e. a method that allows determining the quantitative content and activity of an α-priori indeterminate antioxidant in a test sample), and the technical effect consists in determining activity of an antioxidant and its true quantitative content (i.e. expressed in standardized, not relative units) in a test sample. This makes it possible to determine promptly the antioxidant quantitative content in a test sample; it does not require using complicated methods of chemical analysis and physical-chemical methods. The above-stated technical effect becomes evident upon carrying out a method of determining amount and/or activity of an antioxidant in a test according to claim 1. In the above-stated method, the chemiluminescence intensity may be determined by a photometer, a fluorimeter, a spectrophotometer, a spectrofluorimeter, or a chemiluminometer. In a preferable embodiment of the method, the reaction mixture is temperature-stabilized in order to improve the method reproducibility while performing photometric measurements. Known chemical compounds may be used as chemiluminescence activators, the compounds having high quantum efficiency, preferably luminol, lucigenin, 9,10-dibromoanthracene, Eu³⁺-tetracycline, chlorophyll a, protoporphyrin IX, bacteriopheophorbide, meso-tetraphenylporphyrin, rhodamine G, or quinolizine-coumarines.

The method of the invention can be used for determining content of highly reactive compounds and compounds having antioxidant activity in cells, tissues, biological fluids, pharmacological and cosmetic compositions, articles of food, beverages, and raw material for food and cosmetic industry.

Each of the inventions described herein may be characterized by the features of any one or more embodiments, when the features of the embodiments are logically and technically compatible with each other and with the features of the invention itself.

It shall be understood that the methods herein are characterized only by those features which are enough for solving the assigned task, accomplishing the purpose, and achieving the technical effect. No mention of all features of the invention is required, when it is obvious for persons skilled in the art that the assigned task can not be solved, and the purpose can not be accomplished in the full scale in absence of these features. Neither generalized features are required to be limited to certain options, when such features should be known for persons skilled in the art and/or may be determined according to a known practice.

As it shall be clear for a person skilled in the art, the above-assigned task is solved by virtue of the whole set of features of the method; and the key feature of the invention, that assures achieving the technical effect is that photometric measurements are performed starting from the instant of initiation of a photochemical reaction and until the instant when enough data is collected for calculating parameters of kinetic equations.

Up to now, it was not known that the results of photometric measurements starting from the instant of initiation of a photochemical reaction for such mixtures match a simplified mathematical model including only three kinetic equations for reactions of generating and eliminating free radicals. It was not known that these results can be used for determining concentrations of components in a mixture in any instant of time and that high accuracy of determining the reaction rate constant for eliminating free radicals by an antioxidant and the initial antioxidant concentration is assured.

As it was found unexpectedly, concentrations of components of a photochemical reaction, in any instant of time, can be presented in a satisfactory manner by chemical kinetic equations characterizing generating and eliminating free radicals, while it is enough to determine parameters of the kinetic equation for the reaction between the source of radicals and the initiator of generating radicals (or for the reaction of disintegration of the source of radicals, depending on the type of the reaction), for describing the process of generating free radicals; and it is necessary to determine parameters of the kinetic equations for the reaction between free radicals and the chemiluminescence activator and for the reaction between free radicals and the antioxidant. When initial concentrations of the source of radicals (and the initiator of generating radicals) and the chemiluminescence activator are known, and the parameters of kinetic equations characterizing generating free radicals and eliminating thereof are known based on the reaction of interacting with the chemiluminescence activator, the degree can be determined, in which the influence of the test sample on the dependence of radiation intensity on the time of reaction is conditional of the amount of the antioxidant contained in the sample and/or activity thereof. The amount of the antioxidant can be determined with a satisfactory accuracy if photometric measurements are performed starting from the instant of initiation of a photochemical reaction (but not with later start, as it can be found in common methods in photometry).

As it will be understood from the above-stated, concentrations of components of a reaction mixture can be calculated via solving a system of differential equations presenting three main processes: (1) generating free radicals and eliminating thereof in concurring reactions of free radicals with (2) the chemiluminescence activator and with (3) the antioxidant. Since initial concentrations of components of the reactions (1) and (2) are known, parameters of corresponding kinetic equations can be calculated by known techniques. The object of this invention is determining values of parameters of the third kinetic equation (i.e. the reaction between free radicals and the antioxidant), namely the reaction rate constant and the initial concentration of the antioxidant in the reaction mixture.

The system of differential equations presenting concentrations of components can be solved using numerical techniques wherein values of the initial concentration of the antioxidant and the reaction rate constant for the reaction between the antioxidant and free radicals are selected based on the best correlation between the equations and the observable dependence of intensity of chemiluminescence on the time of reaction.

In this method, generating free radicals can be provided via any known reaction, given that initial contents of the chemical agents are known. Free radicals may be generated e.g. as a result of interaction of hydrogen peroxide (H₂O₂) and ions of metals having variable valence (Fe, Co), or as a result of thermal decomposition of compounds (e.g. 2,2'-azobis-amidinopropane and 2,2'-azobis-2-methyl-propane-imidamide dihydrochloride), or as a result of exposing compounds to ultraviolet light (see German patent DE 4304728).

The activator is used for increasing light radiation intensity (intensity of luminescence) during chemical reaction. A substance having a higher quantum efficiency than free radicals have may be used as the activator. Using the luminescence activator yields a multiple increase in radiation intensity, thus allowing detecting small amounts of free radicals.

Adding an antioxidant to the reaction mixture causes a decrease in amount of radicals and a corresponding decrease in radiation intensity. When determining amount and activity of antioxidants, radiation intensity of a reaction mixture is considered to be proportional to concentration of free radicals; and the amount of free radicals depends on the rate of generating and eliminating thereof, with use of the antioxidant and with no use of it. Taking into account these assumptions, radiation intensity (and concentration of free radicals) in the end of a certain period of time after reaction initiation is defined by (1) the reaction rate constant (k_{R}) for generating radicals, (2) the reaction rate constant for eliminating free radicals with no use of antioxidants (i.e. the reaction rate constant k_{Lum} for interaction between the activator and radicals, when neglecting other processes), and (3) the reaction rate constant (k_{In}) for eliminating free radicals by the antioxidant.

Radiation intensity under absence of the antioxidant is described by processes (1) and (2). This intensity is proportional to the reaction rate (2), which reaction describes interaction between free radicals and the luminescence activator, since exactly this reaction causes emerging electron-excited products followed by corresponding emitting luminescence quanta. Both reaction rate constants k_{R} and k_{Lum} are selected using numerical techniques such that the calculated dependence would best fit experimentally found dependence, taking into account known initial concentrations of the source of free radicals [AR]₀ and the luminescence activator [Lum]₀.

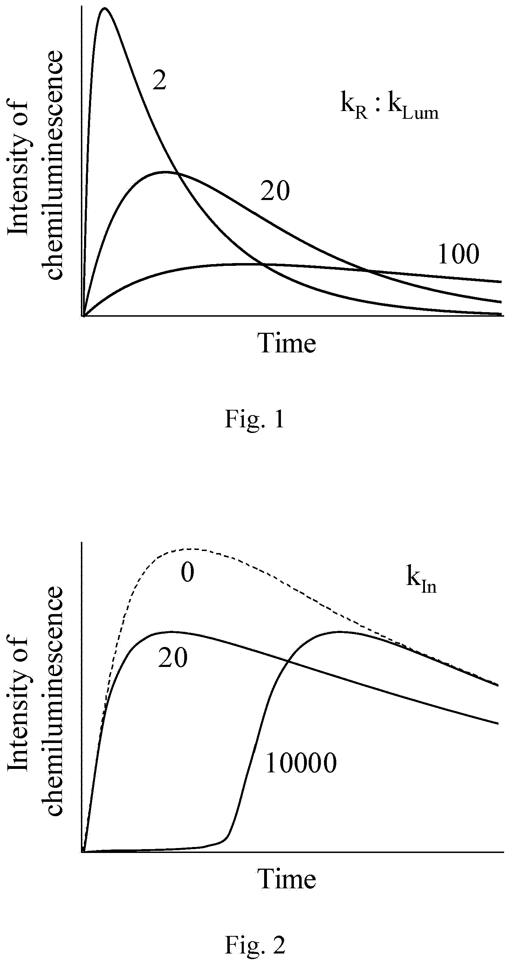

As can be seen in fig. 1, experimentally found dependence of light radiation intensity on the time of reaction under absence of the antioxidant is a two-phase curve consisting of the phase of intensity growth associated with the increase in the rate of generating radicals, and the phase of intensity fall associated with the decrease in the concentration of the source of radicals (the initiator of generating radicals) or the luminescence activator. Two chemical reactions are enough for describing such a two-phase kinetics, and there is only one set of the rate constant values for these reactions, the set being relevant to any kinetic alternative. Thus, the values of the reaction rate constants (1) and (2) under absence of the antioxidant can be identically ascertained via experiment, based on determined dependence of radiation intensity on the time of reaction.

Under presence of the antioxidant, dependence of light radiation intensity on the time of reaction changes and in order to satisfactory forecast concentration of components in a reaction mixture for any instant of time, it is necessary to take into account the influence of the reaction (3) on the rate of the reaction (2) and on the visible radiation intensity (fig. 2). In order to do that, the initial concentration [In]₀ of the antioxidant and the reaction rate constant (k_{In}) for interaction between the antioxidant and the radical are have to be determined. Calculated value of the initial concentration [In]₀ of the antioxidant characterizes the absolute amount of free radicals, that can be bound with the antioxidant of the test sample, while the calculated reaction rate constant (k_{In}) characterizes the amount of free radicals, that can be bound with the antioxidant under its concentration in the solution of 1 mole per liter (i.e. k_{In} is a measure of antioxidant activity of the test sample).

The activator and the antioxidant compete each other for free radicals in the reactions (2) and (3). As it can be seen in fig. 2, "strong" antioxidants having k_{In} much more than k_{Lum} totally forestall and catch free radicals before they would react with the activator, causing technically total suppression of chemiluminescence in the initial period of the reaction, which is called a "latent period" (fig. 2, value "10000"). Once the antioxidant is totally exhausted by reaction with free radicals, the latent period is rapidly replaced by a sharp increase of radiation intensity. "Weak" antioxidants having k_{In} similar to k_{Lum} are not able to react with all or with the most part of radicals before they would react with the activator, therefore just minor decrease of radiation intensity without any latent period can be seen in the initial period (fig. 2, value "20").

Initial concentration of the antioxidant defines duration of the period during which the antioxidant maintains its influence on the dependence of light radiation intensity on the time of reaction. Change in initial concentration of a "strong" antioxidant causes change in duration of the latent period (fig. 3), whereas influence of a "weak" antioxidant remains for a long period of time, exhibiting itself via change in the degree of suppression of radiation intensity (fig. 4). Though only terminal cases are described herein, relating "strong" and "weak" antioxidants, intermediate options undoubtedly exist also, which options are characterized by a mixed influence (fig. 7).

Following to what was said above, the initial concentration and the reaction rate constant affect the reaction (2) in different manner, and there is one and only one combination of values of the initial concentration [In]₀ of the antioxidant and the reaction rate constant (k_{In}) for interaction between the antioxidant and a free radical, for any type of the dependence of light radiation intensity for the reaction mixture on the time of reaction under presence of the antioxidant. This is why the indicated parameters can be identically ascertained, based on experimentally found dependence of light radiation intensity for the reaction mixture on the time of reaction.

Possibility of characterizing influence of an antioxidant on chemiluminescence kinetics by a single kinetic equation for the reaction between the antioxidant and a radical allows characterizing antioxidant properties independently of exact mechanism of reaction in a free-radical process.

What was stated above needs some explanations regarding certain situations. First, there are radical generation systems in which initial amount of a source of radicals does not correspond to the overall amount of generated radicals. For example, one molecule of a source of radicals may decompose into a few radicals or vice versa, one radical can be generated by a few molecules of a source. In such cases a stoichiometric factor n should be used in the reaction (1) of generating radicals, the factor presenting the number of radicals generated by one molecule of the source AR of radicals.

AR → nR•

The stoichiometric factor n can possess any positive value, including fractional one. Using incorrect value of the stoichiometric factor or absence thereof while the factor is required will cause erroneous quantitative determination of antioxidants.

It is supposed that the antioxidant acts one time and totally loses its ability of binding free radicals (i.e. becomes inactive) after interaction with a radical. Many known antioxidants act exactly this way.

### List of Figures

Fig. 1 represents the dependence of chemiluminescence intensity on the time of reaction, under different ratio of the reaction rate constants k_{R} and k_{Lum} (the numbers near plots) in absence of antioxidants.
Fig. 2 represents the dependence of chemiluminescence intensity on the time of reaction, under different values of the reaction rate constant k_{In} (the numbers near plots) in presence of an antioxidant.

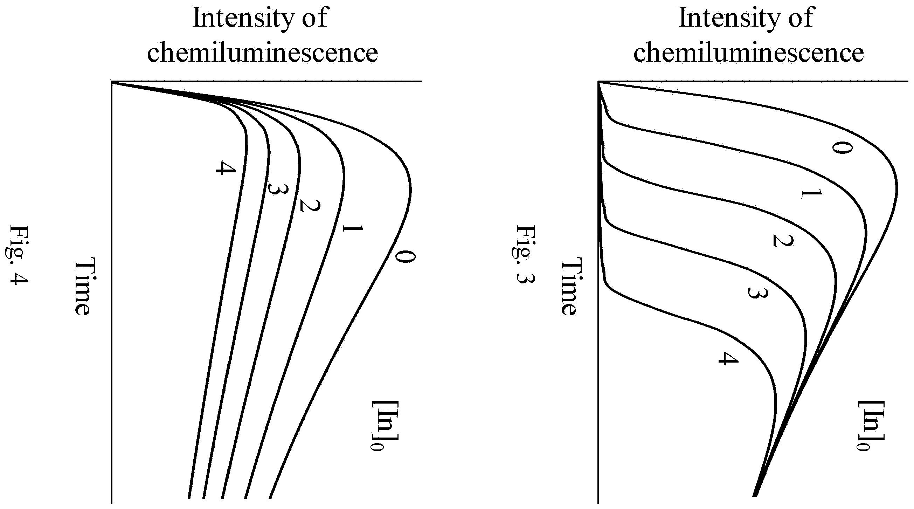

Fig. 3 represents the influence of initial concentration [In]₀ of a "strong" antioxidant (the numbers near plots) on the dependence of chemiluminescence intensity on the time of reaction.
Fig. 4 represents the influence of initial concentration [In]₀ of a "weak" antioxidant (the numbers near plots) on the dependence of chemiluminescence intensity on the time of reaction.

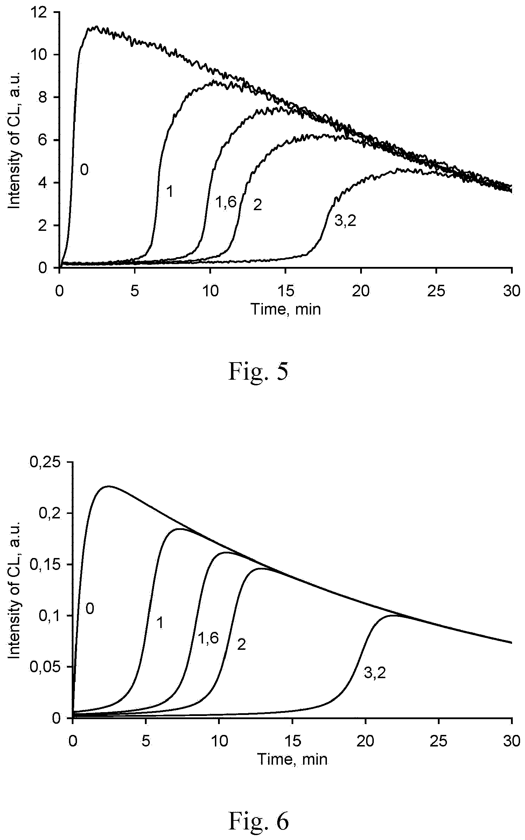

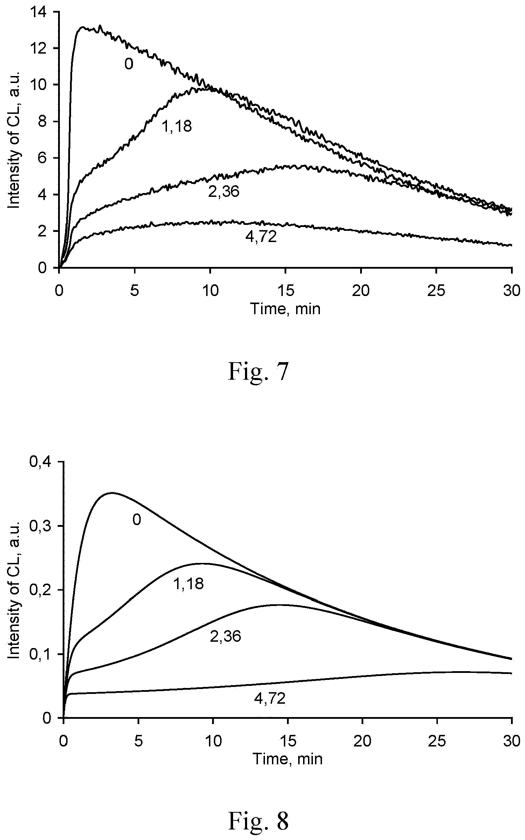

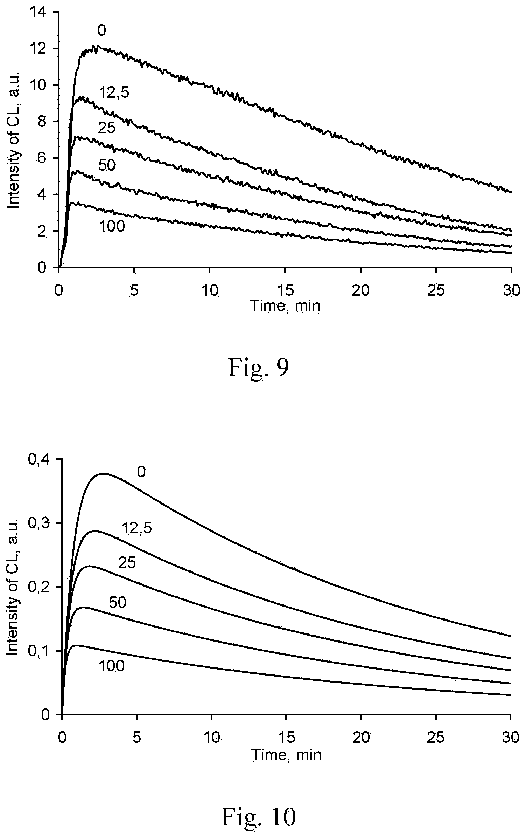

Figs. 5 to 10 represent the influence of antioxidants of trolox (figs. 5 and 6), tocopherol (figs. 7 and 8), and mexidol (figs. 9 and 10) on the dependence of chemiluminescence intensity on the time of reaction (figs. 5, 7, and 9 represent experimental data; figs. 6, 8, and 10 represent results of mathematical simulation). The initial concentration of the antioxidant (expressed in mM) is presented by the numbers near plots.

### Information Confirming a Possibility of Carrying out Invention

The following examples are given only as illustrations of the fundamental principles of the invention; nothing in this part of description can be interpreted as limitation of the scope of claims.

In order to determine prognostic ability of the method described herein, influence of three antioxidants, trolox, tocopherol, and mexidol on chemiluminescence of H₂O₂-CytC system (cytochrome C) has been studied. Experimental data is shown in figs. 5, 7, and 9, particularly the figures show curves presenting the dependence of light radiation intensity of reaction mixtures on the time of reaction.

Under absence of the antioxidant, the curve of light radiation intensity for a reaction mixture is a two-phase curve consisting of a "quick" phase of the radiation intensity increase and a "slow" phase of the radiation intensity decrease. Adding antioxidants into reaction mixtures causes some decrease in chemiluminescence intensity, which decrease depends on the initial concentration of the antioxidant. As it can be seen while comparing figs. 5, 7, and 9, the nature of influence of the antioxidant on the form of curves characterizing dependence of radiation intensity on the time of reaction, is different for the studied antioxidants.

As it can be seen in fig. 5, adding trolox into H₂O₂-CytC system generating radicals causes technically total suppression of chemiluminescence in the beginning of reaction i.e. occurrence of a latent period. The higher initial concentration of trolox, the longer duration of the latent period is.

As it can be seen in fig. 9, adding mexidol into H₂O₂-CytC system generating radicals causes partial suppression of chemiluminescence during the whole period of observation. The degree of chemiluminescence suppression is proportional to initial concentration of mexidol.

As it can be seen in fig. 7, adding tocopherol into H₂O₂-CytC system generating radicals causes effect which can be characterized as a combination of effects attained by adding trolox and mexidol.

Therefore, antioxidants of different nature have different effect on the shape of chemiluminescence curves, thus no any general geometrical parameter can be used for characterizing amount or activity of antioxidants of different chemical nature.

This is exactly why the previous art methods based on measuring duration of the latent period, degree of luminescence suppression, or inclination angle of the initial part of a chemiluminescence curve, do not provide comparable indexes of antioxidant activity for antioxidants of different chemical nature, even when such an activity is expressed using units of activity of a reference antioxidant.

As it can be seen in figs. 6, 8, and 10, parameters of kinetic equations interpreting the form of experimentally found chemiluminescence curve in a satisfactory manner can be selected using numerical techniques for all examples described above.

The form of chemiluminescence curve under absence of antioxidants is simulated by two chemical reactions in which generating free radicals (R•) from hydrogen peroxide (H₂O₂) and interaction thereof with luminol (Lum) occur:

(1) H₂O₂ → R•

(the reaction rate constant is denoted by k_{R})

(2) R• + Lum → reaction (2) products

(the reaction rate constant is denoted by k_{Lum}).

The form of chemiluminescence curve under presence of an antioxidant is simulated, taking into account that the third reaction of interaction between a free radical (R•) and the antioxidant (In) additionally occurs concurrently with the second reaction between free radicals and luminol:

(3) R• + In → reaction (3) products

(the reaction rate constant is denoted by k_{In}).

In the above-described examples, initial values of concentration of hydrogen peroxide, luminol, and antioxidants were known in advance: [H₂O₂]₀ = 10 µM, [Lum]₀ = 25 µM. The concentration [In]₀ is presented by the numbers near corresponding curves in figs. 4 to 9.

Calculated values of the constant rates of the reactions (1) and (2) under absence of antioxidants were k_{R} = 0,04 ± 0,01 µM per minute, k_{Lum} = 0,05 ± 0,01 µM per minute. Calculated value of the constant rate k_{In} of the reaction between the antioxidant and a free radical (reaction (3)) was 60 ± 10 µM per minute for trolox, 2 ± 0,5 µM per minute for tocopherol, and 0,04 ± 0,01 µM per minute for mexidol. The calculated values match the actual ones.

Thus, following from the above, the claimed method allows determining actual initial concentration of an antioxidant in a reaction mixture.

Moreover, following from comparison of figs. 5, 7, and 9, the value of the reaction rate constant for interaction between an antioxidant and a radical can be used as a general measure of antioxidant activity of any substance, independently of the chemical nature thereof. The reaction rate constant allows forecasting the nature of influence of an antioxidant having a known initial concentration on behavior of free-radical processes in a satisfactory manner, particularly, it allows calculating the period of protection action and the oxidation level for components of the reaction mixture, depending on duration of the free-radical process and so on.

The above description shall only be considered as illustrative one, not intended for description of all and/or each of possible embodiments or options of carrying out the inventions, since such a description would be impracticable.

The inventions described above can be implemented and/or carried out by different ways, including by replacing essential features thereof with equivalents which are already known or may become known in the future. Newly created equivalent solutions will still be protected by the following claims.

## Claims

1. A method of determining amount and/or activity of an antioxidant in a test sample, the method including:
performing photometric measurements of a reaction mixture having known initial concentrations of a source of free radicals ([AR]₀) and a chemiluminescence activator ([Lum]₀), starting from the instant of reaction initiation;
selecting a reaction rate constant (k_{R}) for generating free radicals and a reaction rate constant (k_{Lum}) for eliminating free radicals upon interacting with the activator, based on dependence of light radiation intensity of the mixture on the time of reaction by using numerical techniques such that the calculated dependence best fits experimentally found dependence, taking into account known initial concentrations of the source of free radicals [AR]₀ and the luminescence activator [Lum]₀;
preparing a new reaction mixture having known values of [AR]₀ and [Lum]₀ and containing an antioxidant;
performing photometric measurements of the new mixture, and
taking into account the known values of [AR]₀, [Lum]₀, k_{R}, and k_{Lum}, basing on change in chemiluminescence intensity of the new reaction mixture, depending on duration of the period of time passing from the instant of reaction initiation, determining an initial concentration [In]₀ of the antioxidant and a reaction rate constant (k_{In}) for interaction between free radicals and the antioxidant by using numerical techniques based on the best correlation between the equations and the observable dependence of intensity of chemiluminescence on the time of reaction.

2. The method of claim 1, wherein the chemiluminescence intensity is determined by a photometer, a fluorimeter, a spectrophotometer, a spectrofluorimeter, or a chemiluminometer.

3. The method of claim 1, wherein the reaction mixture is temperature-stabilized.

4. The method of claim 1, wherein a known chemical compound having high quantum efficiency may be used as the chemiluminescence activator, preferably selected from a group consisting of luminol, lucigenin, 9,10-dibromoanthracene, Eu³⁺-tetracycline, chlorophyll a, protoporphyrin IX, bacteriopheophorbide, meso-tetraphenylporphyrin, rhodamine G, and quinolizine-coumarines.

5. Use of the method of claim 1 for determining content of reactive compounds in cells, tissues, and biological fluids.

## Patentansprüche

1. Verfahren zur Bestimmung der Menge und/oder der Aktivität eines Antioxidans in einer Testprobe, wobei das Verfahren umfasst:
die Durchführung von den photometrischen Messungen eines Reaktionsgemisches mit bekannten Anfangskonzentrationen einer Quelle für freie Radikale ([AR]₀) und eines Chemilumineszenz-Aktivators ([Lum]₀), ab dem Zeitpunkt der Initiierung einer Reaktion;
die Auswahl einer Reaktionsgeschwindigkeitskonstante (k_{R}) zur Erzeugung freier Radikale und einer Reaktionsgeschwindigkeitskonstante (k_{Lum}) zur Eliminierung freier Radikale unter gegenseitiger Beeinflussung mit dem Aktivator, in Abhängigkeit der Intensität der Lichtemission des Gemisches von der Reaktionszeit mit numerischen Verfahren, so dass die berechnete Abhängigkeit am besten der experimentell bestimmten Abhängigkeit entspricht,
unter Beachtung der bekannten Anfangskonzentrationen einer Quelle für freie Radikale [AR]₀ und des Chemilumineszenz-Aktivators [Lum]₀;
die Herstellung eines neuen Reaktionsgemisches mit bekannten Werten, [AR]₀ und [Lum]₀, und mit einem Antioxidans;
die Durchführung von den photometrischen Messungen des neuen Gemisches, und
unter Beachtung der bekannten Werte [AR]₀, [Lum]₀, k_{R}, und k_{Lum}, basierend auf einer Änderung der Intensität der Chemilumineszenz des neuen Reaktionsgemisches, in Abhängigkeit von der Laufzeit des Zeitraumes von dem Zeitpunkt der Initiierung der Reaktion - die Bestimmung einer Anfangskonzentrationen [In]₀ des Antioxidans und einer Reaktionsgeschwindigkeitskonstante (k_{In}) für die gegenseitige Beeinflussung zwischen freien Radikalen und dem Antioxidans mit numerischen Verfahren basierend auf der besten Korrelation zwischen den Gleichungen und der beobachteten Abhängigkeit der Intensität der Chemilumineszenz von der Reaktionszeit.

2. Verfahren nach Anspruch 1, wobei die Intensität der Chemilumineszenz durch ein Photometer, ein Fluorometer, ein Spektrophotometer, ein Spektrofluorometer oder ein Chemiluminometer bestimmt wird.

3. Verfahren nach Anspruch 1, wobei das Reaktionsgemisch thermostatisiert wird.

4. Verfahren nach Anspruch 1, wobei eine bekannte chemische Verbindung mit hoher Quantenausbeute als Chemilumineszenz-Aktivator, vorzugsweise ausgewählt aus der Gruppe bestehend aus Luminol, Lucigenin, 9,10-Dibromanthracen, Eu3⁺-Tetracyclin, Chlorophyll a, Protoporphyrin IX, Bakteriopheophorbid, Meso-Tetraphenylporphyrin, Rhodamin G und Chinolizin-Kumarin, verwendet werden kann.

5. Verwendung des Verfahrens nach Anspruch 1 zur Bestimmung des Gehalts von reaktiven Verbindungen in Zellen, Geweben und biologischen Flüssigkeiten.

## Revendications

1. Procédé de détermination de la quantité et/ou l'activité d'un antioxydant dans un échantillon analysé, le procédé comprenant:
réalisation des mesures photométriques du mélange reactionnel ayant concentrations initiales connues d'une source de radicaux libre ([AR]₀) et d'un activateur de chimiluminescence ([Lum]₀) à partir du moment de l'initiation de la réaction;
sélection de constante de vitesse de la réaction (k_{R}) de la génération des radicaux libres et de constante de vitesse de la réaction (k_{Lum}) d'élimination des radicaux libres lors de l'interaction avec l'activateur, basée sur la dépendance de l'intensité du rayonnement lumineux du mélange du temps de réaction en utilisant méthodes numériques, tels que la dépendance calculée correspond le mieux à la dépendance trouvée à titre expérimental, en tenant compte des concentrations initiales connues de la source de radicaux libre ([AR]₀) et du activateur de chimiluminescence ([Lum]₀);
préparation d'un nouveau mélange reactionnel ayant valeurs connues de [AR]₀ et [Lum]₀ et contennant un antioxydant;
réalisation des mesures photométrique du nouveau mélange, et
compte tenu des valeurs connues de [AR]₀, [Lum]₀, k_{R} et k_{Lum} sur la base du changement en intensité de chimiluminescence du nouveau mélange reactionnel selon la période du temps passant du moment de l'initiation de la réaction, détermination d'une concentration initiale [In]₀ du antioxydant et d'une constante de vitesse de la réaction (k_{In}) pour l'interaction entre les radicaux libres et l'antioxydant en utilisant méthodes numériques, basés sur la meilleure corrélation entre équations et la relation observée entre l'intensité de la chimiluminescence et le temps de réaction.

2. Procédé selon la revendication 1, dans lequel l'intensité de chimiluminescence est déterminée par un photomètre, un fluorimètre, un spectrophotomètre, un spectrofluorimètre ou un chemiluminomètre.

3. Procédé selon la revendication 1, dans lequel le mélange reactionnel est thérmostabilisé.

4. Procédé selon la revendication 1, dans lequel un composé chimique connu ayant une efficacité quantique élevée peut être utilisé comme l'activateur de chimiluminescence, choisi de préférence parmi le group constitué de luminol, lucigenin, 9,10-dibromoanthracène, Eu³⁺-tétracycline, chlorophylle a, protoporphyrine IX, bactériophéophorbide, meso-tétraphénylporphyrine, rhodamine G et quinoléizine-coumarines.

5. Utilisation du procédé selon la revendication 1 pour la détermination de teneur des composés réactifs dans cellules, tissus et fluides biologiques.
